# EUROPEAN PATENT APPLICATION

(11) **EP 2 280 361 A2**
(43) Date of publication of application: **02.02.2011**
(21) Application number: 10170710.7
(22) Date of filing: 23.07.2010
(51) Int. Cl.: G06F 19/00, A61B 5/044

(54) **Medical data display with 3-D and 2-D color mapping**

(30) Priority: 24.07.2009 US 460796
(71) Applicant: Cardiac Science Corporation, Bothell, WA 98021 (US)
(72) Inventor: Luo, Shen, Bothell, WA 98021 (US)
(74) Representative: Geyer, Werner

(57) **Abstract**

A device and method for displaying medical data for a stress test monitoring system. A computer-implemented method and apparatus for displaying electrical impulse data of a human heart for analysis includes receiving cardiac electrical impulse data produced during a cardiac stress test by a plurality of electrode leads adapted for placement on a patient and calculating parameters for a plurality of display windows based on the data and providing a main display in which the plurality of display windows are positioned. A three dimensional color mapping plot is displayed in one of the plurality of display windows and a two dimensional color mapping plot is displayed in another of the plurality of display windows. Additionally, a plot of raw lead data from at least one lead is displayed in one of the plurality of display windows.

## Description

### FIELD OF THE INVENTION

The present invention relates to medical device displays and methods for presenting patient data. More particularly, the present invention relates to communicating cardiac patient data including use of a color gradient mapping instrument that produces an enhanced display for monitoring and diagnosis including 3-D and 2-D color mapped graphs.

### BACKGROUND OF THE INVENTION

The ability to quickly and effectively diagnose and treat cardiac conditions has been widely desired in the medical community for many decades. The American Heart Association has estimated in recent years that approximately eighty million people in the United States alone have one or more forms of cardiovascular disease. Effective tests used to help identify and treat cardiac conditions are accordingly very important to physicians and members of the medical community around the world.

One common way in which patients are monitored and tested for some heart related conditions is through use of a cardiac stress test. A cardiac stress test is used to determine arterial blood flow to the heart during exercise.

A typical treadmill stress test is performed by bringing a patient to an exercise laboratory where he or she can be observed by a physician or medical specialist. Resting heart rate and blood pressure are recorded, and electrodes are attached to the chest and torso of the patient. In the past, a twelve lead electrocardiogram ("EKG" or "ECG") has been typically used where each lead of the ECG represents a different portion of the heart, with adjacent leads indicating a single wall. In some common configurations, leads aVL and I represent the superior or top and outer left portion of the heart, leads II, aVF, and III represent the inferior portion or bottom of the heart, leads V1 and V2 represent the septum or partition of the heart, and leads V3 and V4 represent the anterior or front portion of the heart. The treadmill is started at a slow speed and at little or no inclination. The speed and slope of the treadmill is then increased every three minutes or as otherwise required by the stress test protocol. ECG readings are then displayed on a monitor and may be recorded.

In such a test, structures on an ECG such as QRS complexes, ST level, ST slope, wave and interval lengths, and various other information received from leads are measured and observed by a physician or medical professional in order to make a diagnosis or medical determination. Historically, ST level and slope numerical measurements have been presented in a variety of formats during stress exercise testing, monitoring, and reporting.

In some past stress presentations, a collection of screens filled with raw lead data from a plurality of leads was generally displayed for review. Although these system presentations provided access to some heart data for comparison, (such as median complex, ST level, ST slope, worst case ST depression graphs, etc.) this kind of presentation required comparing and piecing together bits of data from a large number of plots, display screens, or printouts. Final reports and trend graphs that were generated contained data where data significance was not always as readily apparent or quickly understandable as desired.

Other past systems were also made, having similar limitations of presentation in their displays as well. In some of these displays, ST level change was presented only as a digital number from a single lead without leads for comparison. Also, views of the ST change history were not present.

In one past device, made by GE Marquette, a Waterfall mapping display was provided which used a 2-D color graph versus time to show ECG beat voltage changes. By using a 2-D color format, the presentation of PQRST peak amplitude color slices, which are tilted into an overhead view and sequentially stacked, indicates multiple segment timing changes during the overall test time. This tends to deemphasize specific amplitude axis changes being shown within only a single lead per graph. In general, stress testing applications, the clinically significant ischemic patterns of ST voltage changes in multiple leads were not presented in a more effective way compared to past devices. Many measurements displayed were not clinically significant for stress testing or were simply redundant.

Therefore, what is needed is a display device which overcomes the deficiencies of the past, and which enables a medical professional to rapidly understand the parameters being measured during a cardiac stress test, cardiac monitoring, or other similar type of test. A device is needed which makes the maximum amount of relevant data readily comprehensible so that an informed diagnosis can be quickly made. Moreover, a display that better promotes synthesis of diagnosis and which accommodates quick review of status data and testing results is desired.

### SUMMARY OF THE INVENTION

The present invention overcomes the problems of the prior art by providing a method and device to efficiently display a large amount of patient data in an easily understandable format using a presentation which enables a medical professional to rapidly make observations and diagnosis based on the information displayed. In one embodiment, a computer-implemented method for displaying electrical impulse data of a human heart for analysis is provided. In this method, data is received from one or more input files, wherein the data is cardiac electrical impulse data produced during a cardiac stress test by a plurality of electrode leads adapted for placement on a patient. The method further includes calculating parameters for a plurality of display windows based on the data and providing a main display in which the plurality of display windows are positioned. The method also includes displaying a three dimensional color plot in one of the plurality of display windows based on the data and the parameters and displaying a two dimensional color plot in one of the plurality of display windows based on the data and the parameters. Further, in the display windows, the two dimensional color map corresponds to the three dimensional color plot. Colors can be used to represent ST level. Additionally the method includes displaying a plot of raw lead data from at least one lead in one of the plurality of display windows.

Further, the three dimensional color plot may be interactively rotated on the main display such that a user may select a desired viewpoint to observe the three dimensional color plot.

The lead data can be shown on the two dimensional color plot and the three dimensional color plot in anatomic lead order.

The color shown in the two dimensional color plot and the three dimensional color plot can represent ST level.

The inventive method can further include a two dimensional color map located adjacent to the two dimensional color plot on the main display, the two dimensional color map having an independent color scale.

The inventive method can further include generating a printable one page stress test report containing the information contained on the main display.

In addition, the inventive method can further include displaying a plot of raw channel data and a representative beat plot in the main display corresponding to a user selected location on the two dimensional plot.

In another embodiment according to the present invention, a medical apparatus for generating a stress test display is included. The medical apparatus includes a data acquisition module adapted for connection to a patient undergoing a cardiac stress test, a monitor capable of real time display of color mapped plots, and a computer. The computer includes a processor and a memory coupled to the data acquisition module and the monitor. The computer contains exercise stress test software. Operation of the software includes means for providing a display for reviewing information obtained during a cardiac stress test and means for receiving data wherein the data is cardiac electrical impulse data produced during the cardiac stress test by a plurality of electrode leads adapted for placement on a patient. Operation of the software further includes means for calculating values for generating plots based on the data, means for displaying a three dimensional color plot in a first portion of the display based on the data and the values, means for displaying a two dimensional color plot in a second portion of the display based on the data and the values, and means for displaying a plot of raw lead data from at least one lead in a third portion of the display. The two dimensional color plot corresponds to the three dimensional color plot.

The inventive medical apparatus can be provided such that the three dimensional color plot may be rotated on the display such that it may be observed from a desired view point.

The lead data can be shown on the two dimensional color plot and the three dimensional color plot in anatomic lead order.

Further, the inventive medical apparatus can be embodied such that the lead data can be shown on the two dimensional color plot and three dimensional color plot in an order which is user selectable. The first portion can be located vertically adjacent to the second portion on the display.

According to an embodiment of the present invention, a computer-implemented method for displaying electrical impulse data of a human heart for analysis includes providing a display for presenting information obtained during a cardiac stress test, receiving data, wherein the data is cardiac electrical impulse data produced during the cardiac stress test by a plurality of electrode leads adapted for placement on a patient, and calculating electrocardiography values for use in plots based on the data. The method also includes displaying a three dimensional color plot in a first portion of the display based on the data and the values and displaying a two dimensional color plot in a second portion of the display based on the data and the values. The two dimensional color plot corresponds to the three dimensional color plot. Additionally, the method includes displaying a plot of raw lead data from at least one lead in a third portion of the display.

The method may be provided such that the three dimensional color plot may be rotated on the display such that a user may select the desired viewpoint of the three dimensional color plot. Further, the lead data can be shown on the two dimensional color plot and three dimensional color plot in anatomic lead order.

In addition, the lead data can be shown on the two dimensional color plot and the three dimensional color plot in an order which is user selectable and/or the three dimensional color plot can be located directly above the two dimensional color plot.

In another embodiment according to the present invention, a computer-implemented method for displaying electrical impulse data of a human heart for analysis includes providing a display for presenting information obtained during a cardiac stress test. The method further includes receiving data, wherein the data is cardiac electrical impulse data produced during the cardiac stress test by a plurality of electrode leads adapted for placement on a patient. The method also includes calculating values for generating plots based on the data, displaying a three dimensional color plot in a first portion of the display based on the data and the values, and displaying a two dimensional color plot directly below the plot located in the first portion of the display, based on the data and the values. The two dimensional color map corresponds to the three dimensional color plot in colors used to depict ST level. The method finally includes displaying a plot of raw lead data from at least one lead located near the center of the display.

In the computer-implemented method the three dimensional color plot may be rotated on the display such that it may be observed from a desired viewpoint. Further, the lead data can be shown on the two dimensional color plot and the three dimensional color plot in anatomic lead order.

The lead data can be shown on the two dimensional color plot and three dimensional color plot in an order which is user selectable. Further, the three dimensional color plot can be located directly above the two dimensional color plot.

In another embodiment, a computer-implemented method for displaying electrical impulse data of a human heart for analysis during a cardiac stress test includes receiving data, wherein the data is cardiac data acquired by measuring the electric potential between various points of the body using a data acquisition module and a plurality of electrode leads adapted for patient attachment. It further includes generating electrocardiogram information for each of the plurality of electrode leads based upon the data and calculating ST level data for each of the plurality of electrode leads throughout the stress test. The method also comprises displaying a three dimensional color plot using a first color scale where the ST level data are represented by color and amplitude for the plurality of electrode leads and displaying a two dimensional color plot using the first color scale where ST level data is represented by color for the plurality of electrode leads. Also, the method of this embodiment includes displaying a two dimensional color map of various electrocardiogram parameters based upon a second color scale, the two dimensional color map located adjacent to the two dimensional color plot such that the two dimensional color plot and the two dimensional color map are positioned to correspond horizontally by time.

In another emobodiment, a medical apparatus for generating a stress test display includes a treadmill for patient use during the course of a cardiac stress test, a plurality of electrode leads adapted for patient attachment during the cardiac stress test, and a data acquisition module adapted for connection to the electrode leads for measuring electrical potential between various body locations on a patient. Further, the medical apparatus includes a monitor capable of presenting a real time display of color mapped plots and a computer, comprising a processor and a memory, coupled to the data acquisition module and the monitor. The computer containing exercise stress test software, where operation of the software includes means for providing a display for reviewing information obtained during a cardiac stress test, means for receiving data, wherein the data is cardiac electrical impulse data produced during the cardiac stress test, means for calculating values for generating plots based on the data, means for displaying a three dimensional color plot in a first portion of the display based on the data and the values, means for displaying a two dimensional color plot in a second portion of the display based on the data and the values, the two dimensional color plot corresponding to the three dimensional color plot, and means for displaying a plot of raw lead data from at least one lead in a third portion of the display.

Yet another embodiment includes a computer readable medium for generating a medical stress test display, including instructions. The instructions directed to providing a display of information obtained during the course of a cardiac stress test, receiving cardiac electrical impulse data for the cardiac stress test, wherein the data is obtained from a data acquisition module connected to electrode leads, and calculating values for generating plots based on the cardiac electrical impulse data. The instructions also directed to displaying a three dimensional color plot in a first portion of the display based on the cardiac electrical impulse data and the values, and displaying a two dimensional color plot in a second portion of the display based on the cardiac electrical impulse data and the values, the two dimensional color plot corresponding to the three dimensional color plot. Further the invention includes instructions directed to displaying a two dimensional color map of various electrocardiogram parameters, the two dimensional color map located adjacent to the two dimensional color plot such that the two dimensional color plot and the two dimensional color map are positioned to correspond horizontally by time.

It will be appreciated that the features mentioned above and those yet to be explained below can be used not only in the indicated combinations but also in other combinations or alone, without departing from the scope of the present invention.

### BRIEF DESCRIPTION OF THE DRAWINGS

The invention may be more completely understood in consideration of the following detailed description of various embodiments of the invention in connection with the accompanying drawings (which also disclose features relevant to the invention), in which:
FIGS. 1a-c illustrate generally examples of prior art display devices.
FIG. 2 illustrates generally an example of a color mapping interface display according to an embodiment of the invention.
FIG. 3 illustrates generally a flowchart of the hardware used to monitor a patient during a stress test according to an embodiment of the invention.
FIG. 4 illustrates generally a flowchart of the phases of a typical stress test according to an embodiment of the invention.
FIGS. 5a-f illustrate generally examples of the mapping interface display at various times during a stress test according to an embodiment of the invention.
FIG. 6 illustrates generally a block diagram setting forth the data flow within the software of the system, according to an embodiment of the invention.
FIG. 7 illustrates generally a diagram of the software generating the output display, according to an embodiment of the invention.
FIGS. 8a-e illustrate generally flowcharts of the software files for generating output displays, according to an embodiment of the invention.

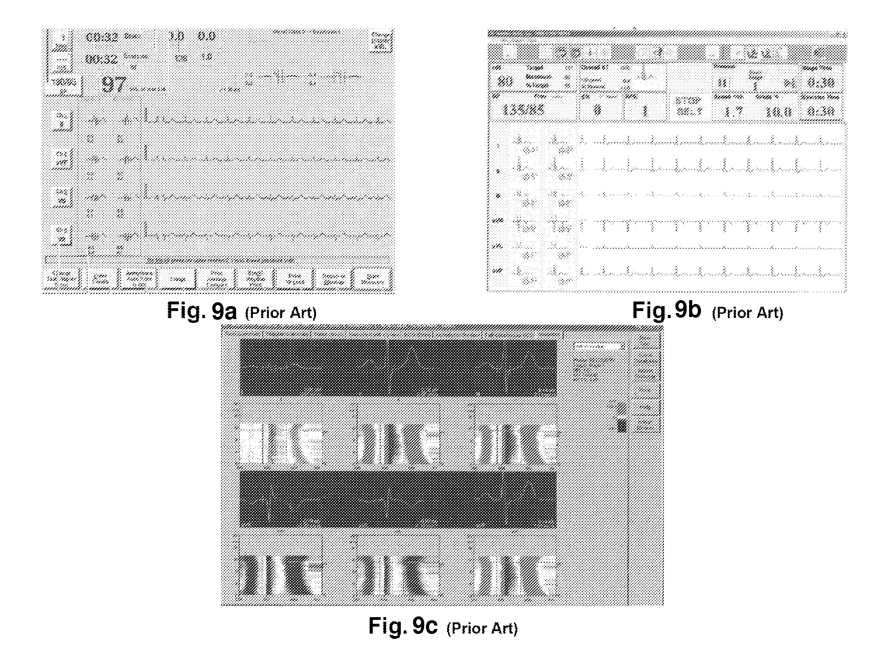

FIGS. 9a-c are color drawings of FIGS. 1a-c.

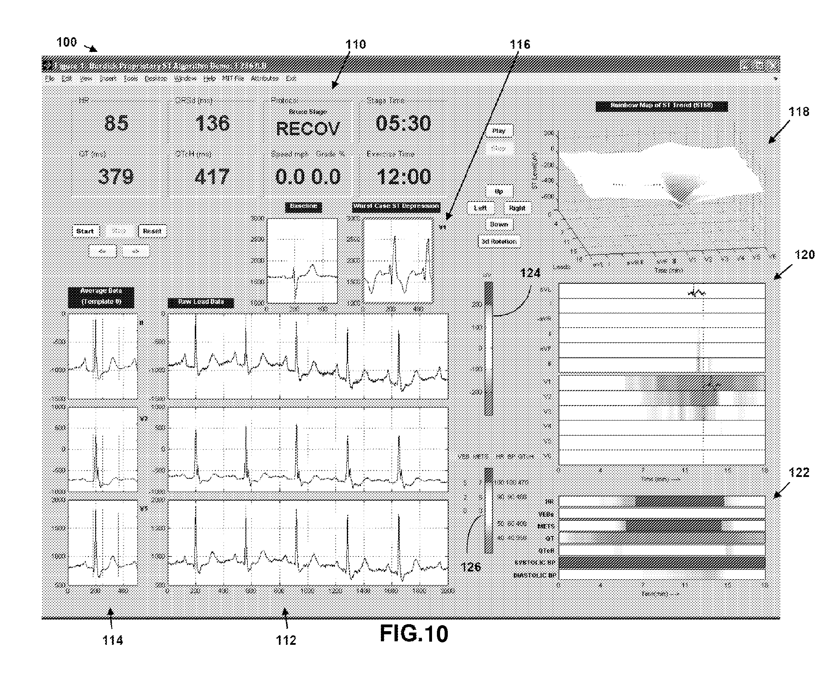

FIG. 10 is a color drawing of FIG. 2.

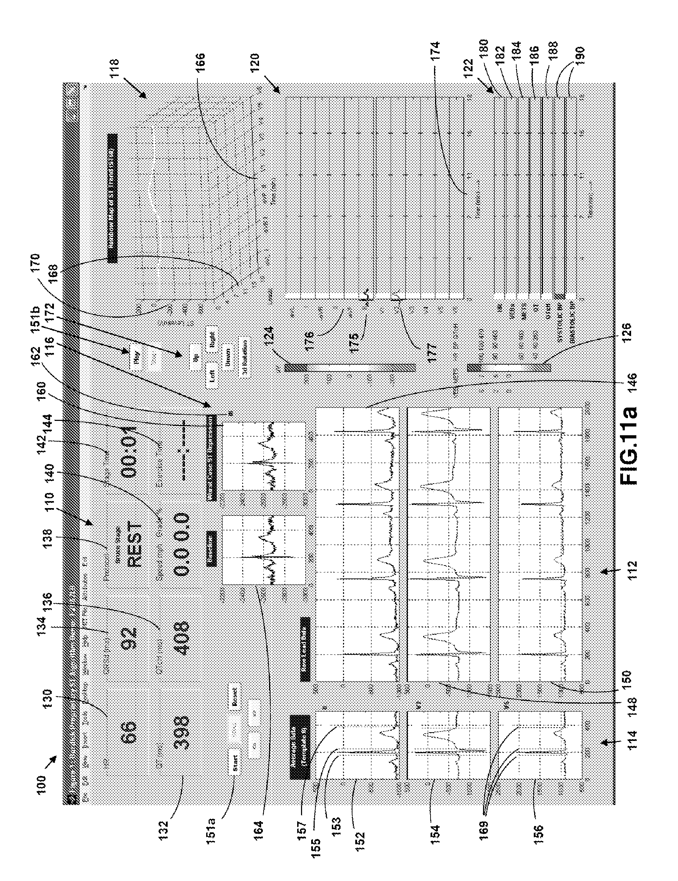

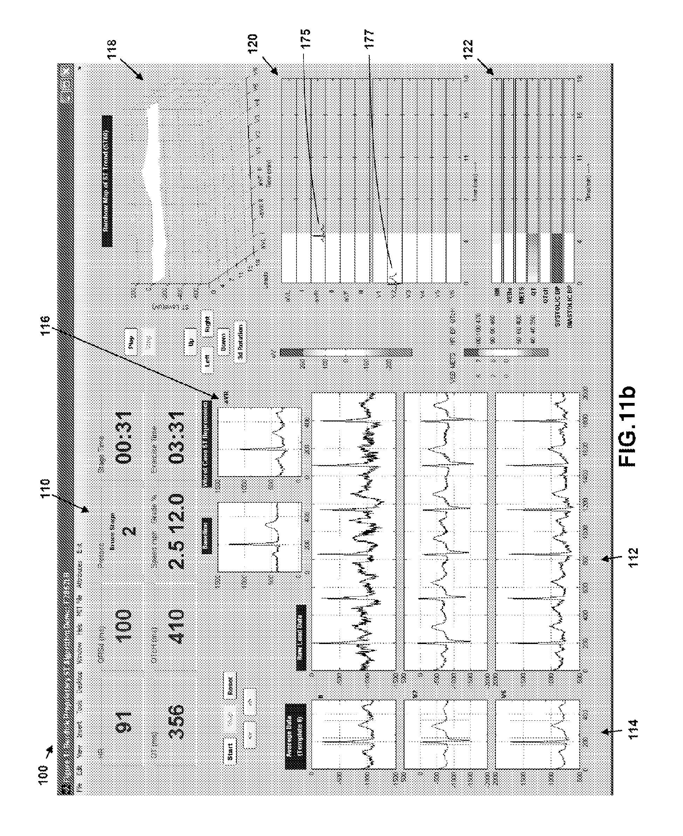

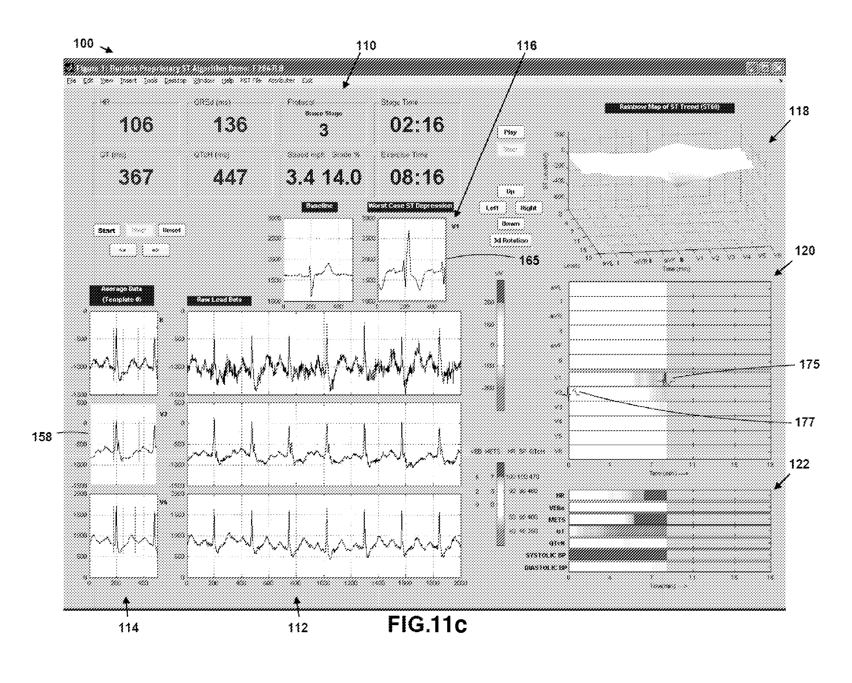

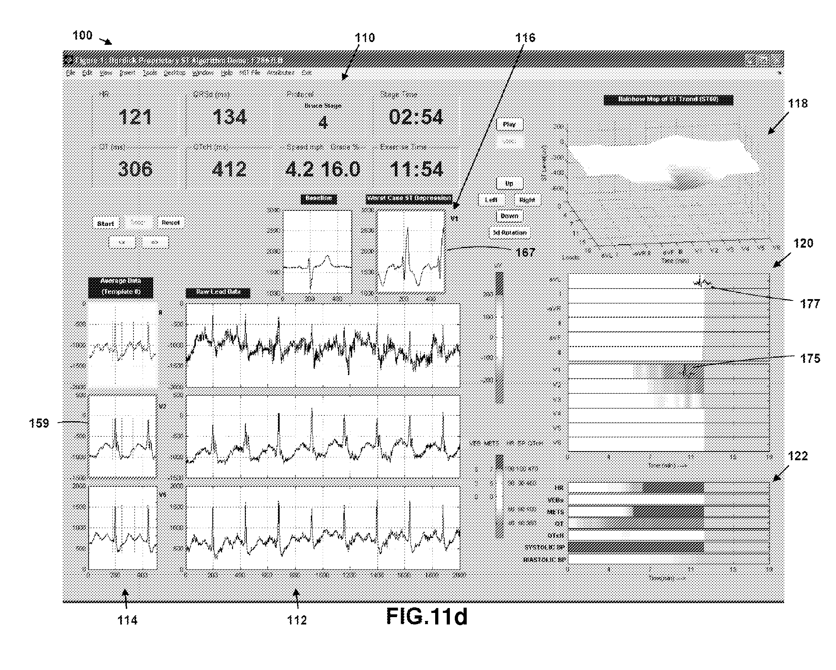

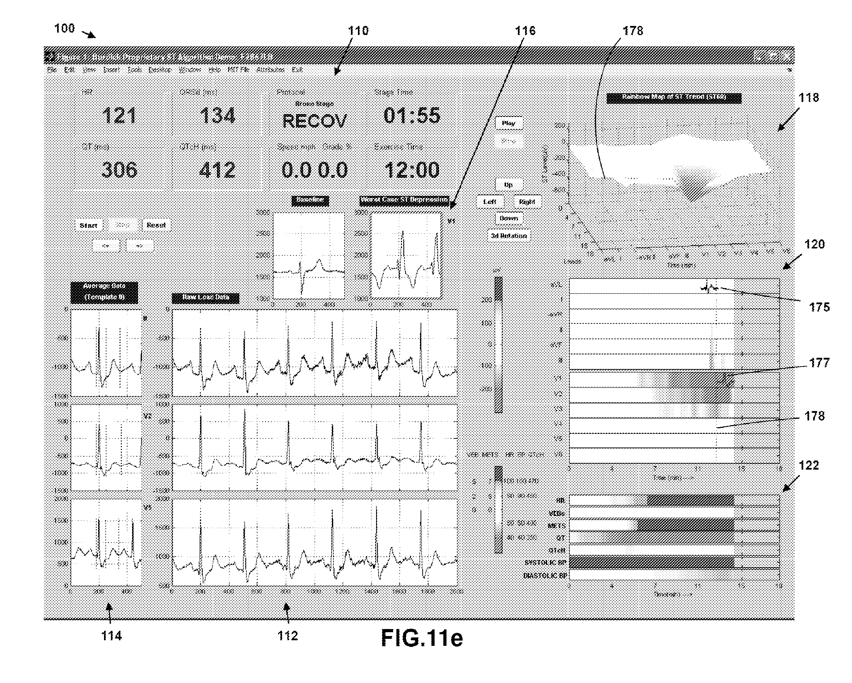

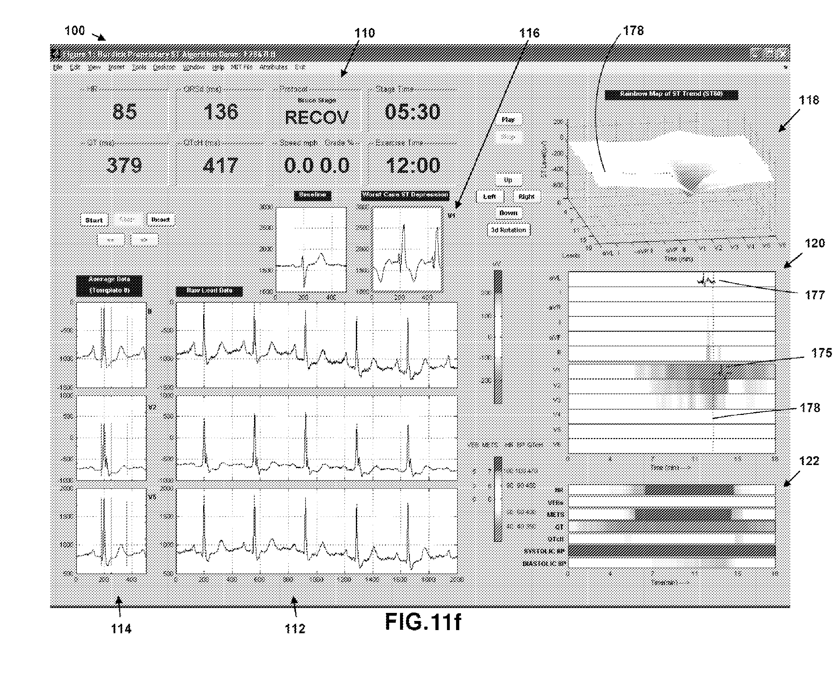

FIGS. 11a-f are color drawings of FIGS. 5a-f.

### DETAILED DESCRIPTION OF THE PREFERRED EMBODIMENTS

The invention may be embodied in other specific forms without departing from the essential attributes thereof, therefore, the illustrated embodiments should be considered in all respects as illustrative and not restrictive.

In various embodiments of this invention an apparatus and method are disclosed for a medical data display. In this display multiple clinical numerical parameters are transformed into an intuitive, clustered color scene group that includes a color 3-D graph with anatomic locations and secondary, supporting two-dimensional time aligned plots/maps which improve measurement, diagnostic accuracy, as well as interpretation time. Both real-time monitoring and offline processing are possible using this display.

The display can readily be used to present data associated with various cardiac monitored events or tests especially related to exercise ECG data, such as a typical 18-minute long biological stress test. During the test, heart beats are gathered for calculation of average beat representations for each of the twelve lead channels measuring ST levels and ST slopes. The data is stored in time sequence order at repeating intervals. A color-mapped display screen is generated allowing a physician reviewer to observe graphical areas highlighting changes throughout the stress testing procedure.

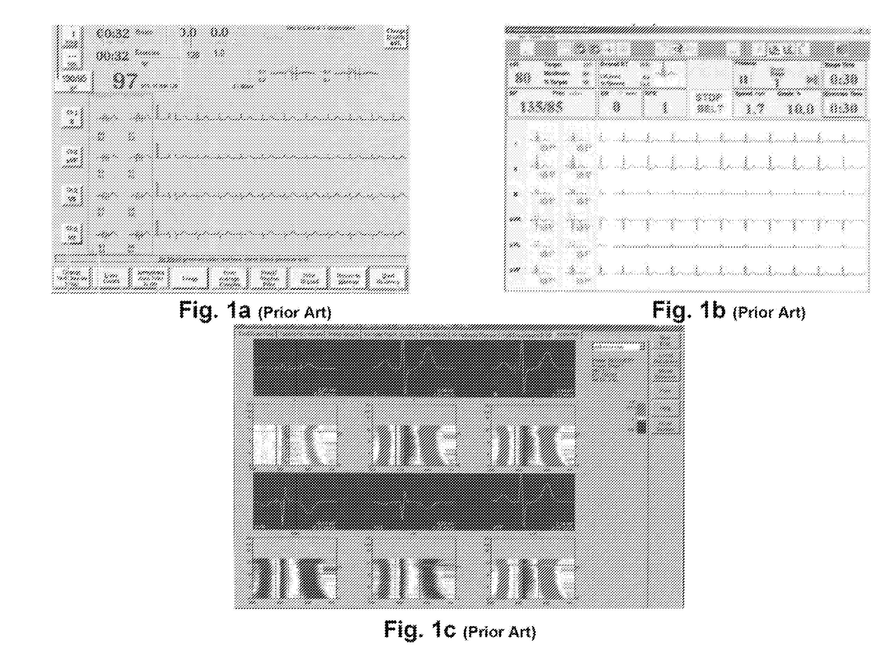

In some past stress system presentations, such as the Burdick stress display seen in Fig. 1a, a collection of screens filled with raw lead data from a plurality of leads was generally displayed for review. Although these system presentations provided access to some limited comparison data and other heart data, (such as median complex, ST level, ST slope, worst case ST trend graphs, etc.) this data required comparing and piecing together bits of data from a large number of plots, display screens, or printouts. Final reports and trend graphs that were generated contained data where data significance was not always as readily apparent or quickly understandable as desired.

Other past systems were also made, such as the Quinton stress display seen in Fig. 1b. Similar limitations of presentation existed in these displays as well. Furthermore, ST level change was presented only as a digital number from a single lead without lead data for comparison and there were no straightforward views of the entire ST change history.

Another past device was made by GE Marquette that provided a Waterfall mapping display which used a 2-D color graph versus time to show ECG beat voltage changes. See Fig. 1c, for example. By using a 2-D color format, the presentation of PQRST peak amplitude color slices, which are tilted into an overhead view and sequentially stacked, indicates multiple segment timing changes during the overall test time. This tends to deemphasize specific amplitude axis changes being shown within only a single lead per graph. In general, stress testing applications, the clinically significant ischemic patterns of ST voltage changes in multiple leads were not presented in a more effective way compared to past devices.

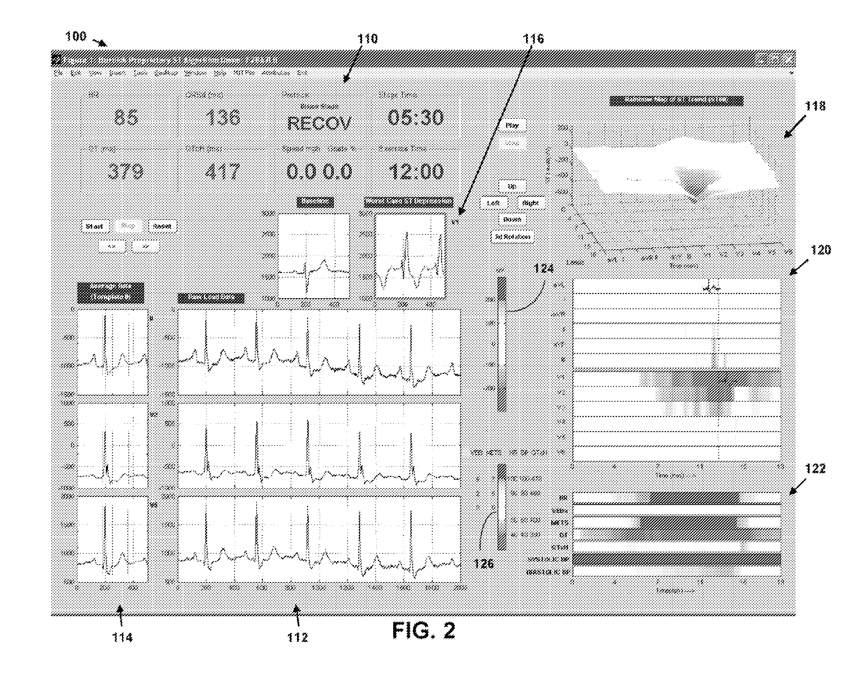

Fig. 2 illustrates generally an example of the color mapping interface display 100 representing an embodiment of the present invention. This example reveals an invention in which the data obtained from a stress test is presented in an easy to understand format. This type of display enables a medical specialist to quickly understand aspects of cardiac activity and diagnose a patient accordingly. The display contains a large amount of general cardiac data and lead data as well as 3-D and 2-D plots. As an overview, these plots and information are grouped around the display screen in an intuitive manner. Plots and information on the left side of the display include a measurement table 110, three channel real-time stress ECG traces 112, three channel average beat plots 114, and a worst lead across twelve leads plot 116 next to the baseline from the same lead. Plots and information on the right side of the display include a 3-D color mapping plot 118, a 2-D color mapping plot 120, key parameters color map bars 122, and color meters 124 and 126. Fig. 2 is presented here as one example of the overall display with data observed approximately eighteen minutes into a cardiac stress test at test completion. A more detailed description of the various components of the display will be set forth later in greater detail.

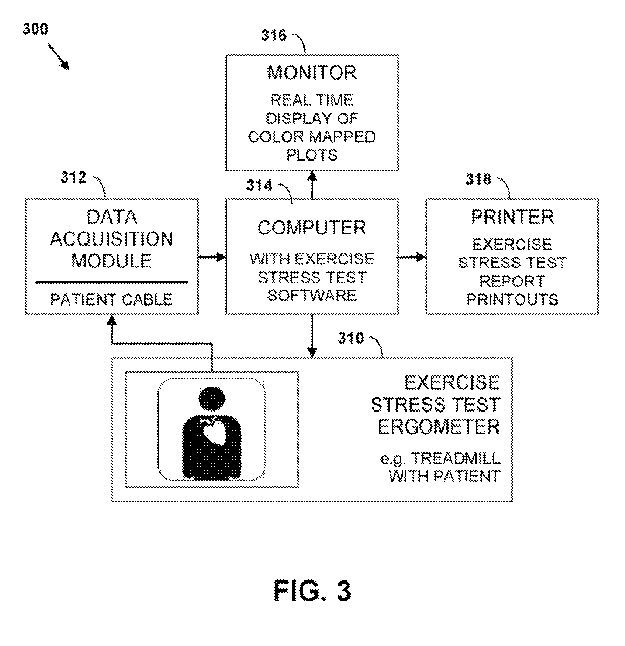

Fig. 3 generally discloses hardware 300 that may be utilized during a stress test conducted on a patient to monitor cardiac activity. When the test is being conducted, a patient typically exercises by walking or running on a treadmill 310. Data regarding the patient's heart is obtained from leads coupled to the patient and is transferred via a cable to the data acquisition module 312. The data from the module 312 is provided to a computer 314 containing a processor and a memory which is loaded with exercise stress test software. As explained later in greater detail, this software reads data, calculates parameters and generates an information intensive display to communicate the data and parameters. A presentation is generated which can be displayed on one or more coupled monitors 316. Additionally, reports may be generated for printing on a printer 318. A one-page compact exercise stress test report printout is possible, compared to the many page stress test report in the traditional printouts. It is understood that various computers, monitors, printers or similar peripheral devices may be readily implemented as part of the system described in this application.

Other embodiments may include use for chemical stress tests or electrical stress tests in place of, or in addition to, an exercise stress test. In a chemical stress test, a medication producing stress is administered intraveneously, often through an intravenous line in the patient's arm. Electrodes attached to the patient sense heart activity while ECG data is recorded. In an electrical stress test, a diseased heart is stimulated using low-voltage electricity via an electrode set. Results obtained from monitoring the resulting heartbeat data are compared to those of a healthy heart.

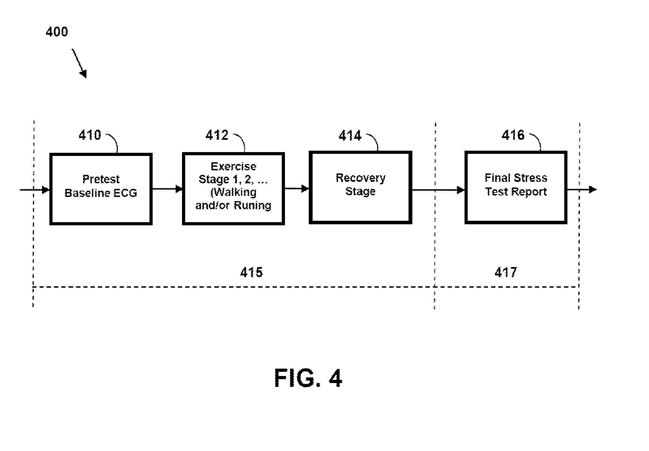

Fig. 4 is a flowchart of the phases of a typical stress test that might incorporate the described display, as set forth as numeral 400. First, at the pretest/baseline stage 410, a Baseline ECG is performed on the patient's heart while in a standing but resting position. Next, the patient undergoes an exercise stage 412 in which the patient walks or runs to subject the patient's heart to increased stress thereby more readily revealing arteries that have blockages or reduced blood flow. Often this involves increasing the slope or inclination of a treadmill (or bicycle workload) at predetermined time intervals. The exercise stage 412 is followed by a recovery stage 414 during which a patient slows movement and decreases his/her heart rate. At each stage, data is acquired and is displayed as part of a realtime output of information related to the patient's heart. Therefore, stages 410, 412, and 414 represent the entire monitoring process 415 of the stress test. The monitoring process and realtime display data is followed by a final stress test report 416. Such a report may either be reviewed immediately by a medical specialist, or may be saved or printed for later review and diagnosis. Generating this report makes up a reporting stage 417 that follows the monitoring process 415.

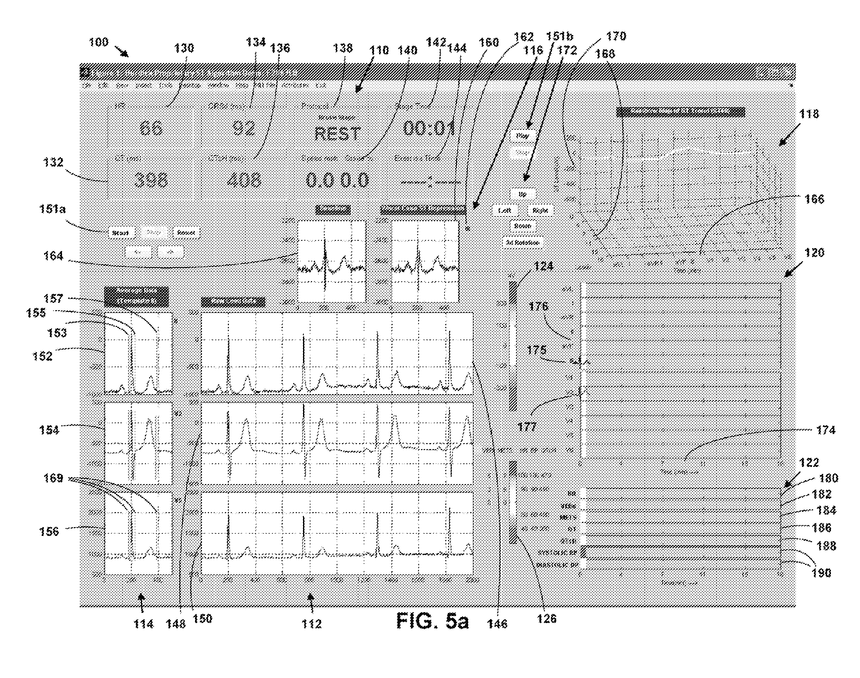

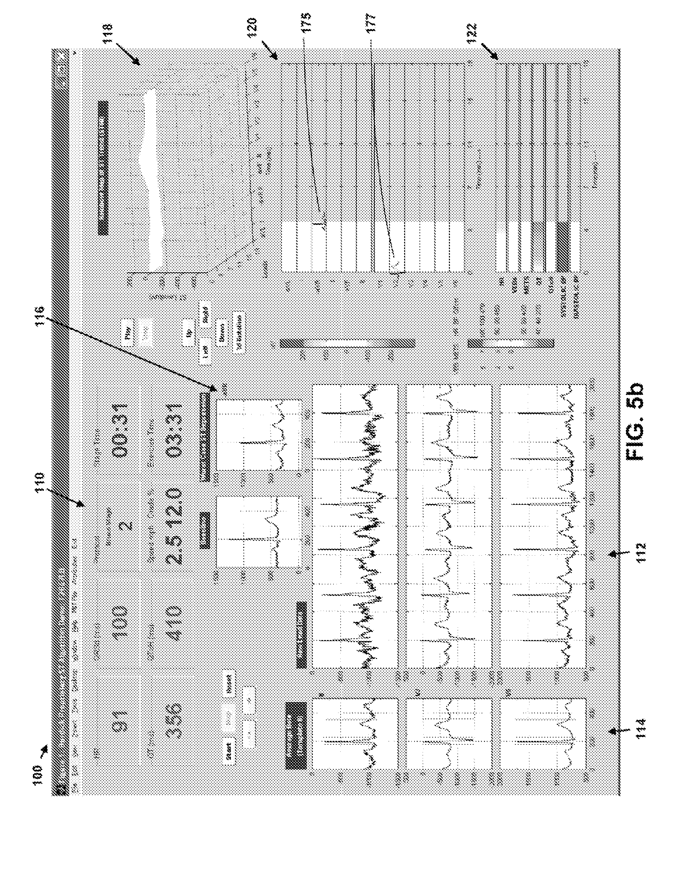

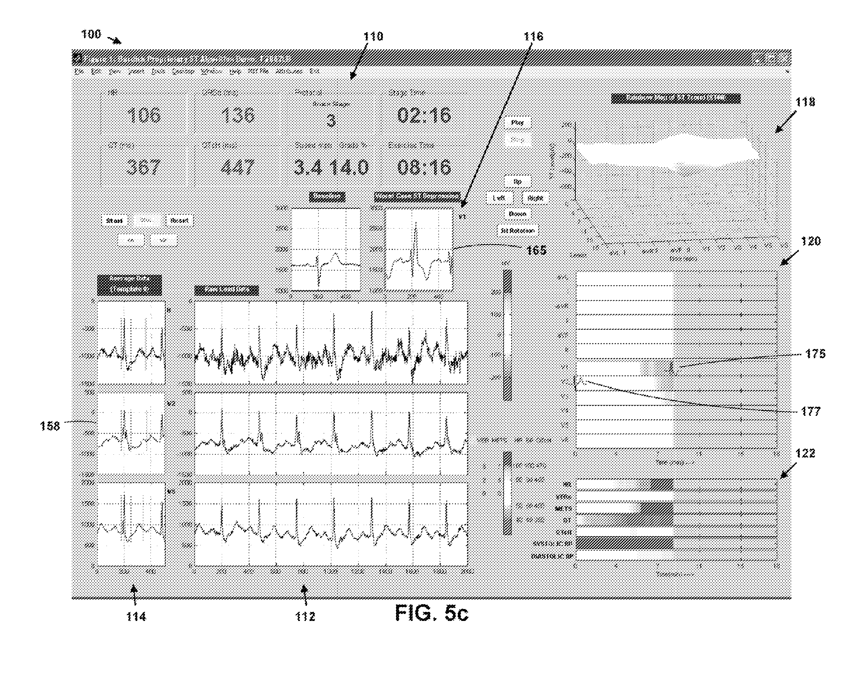

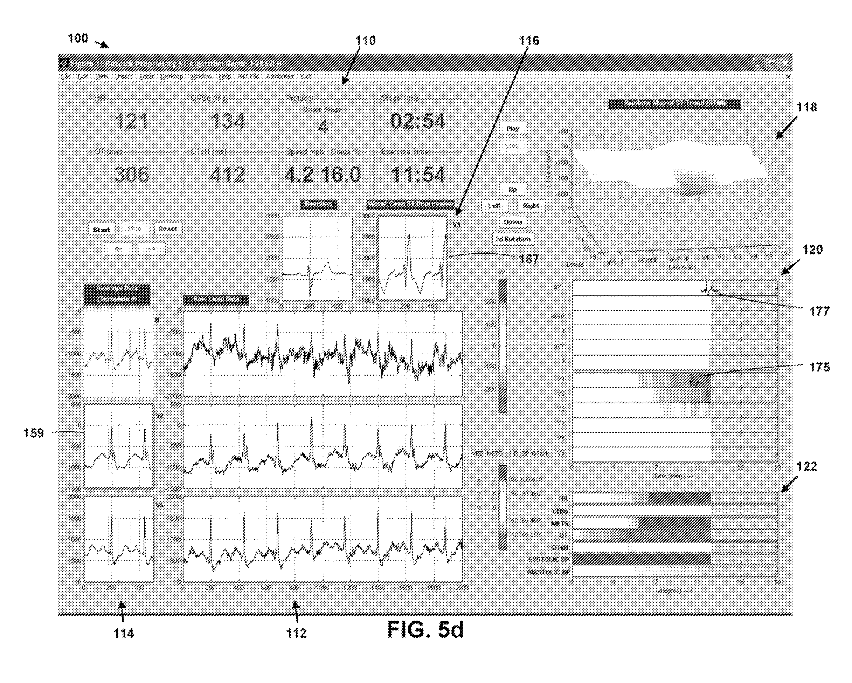

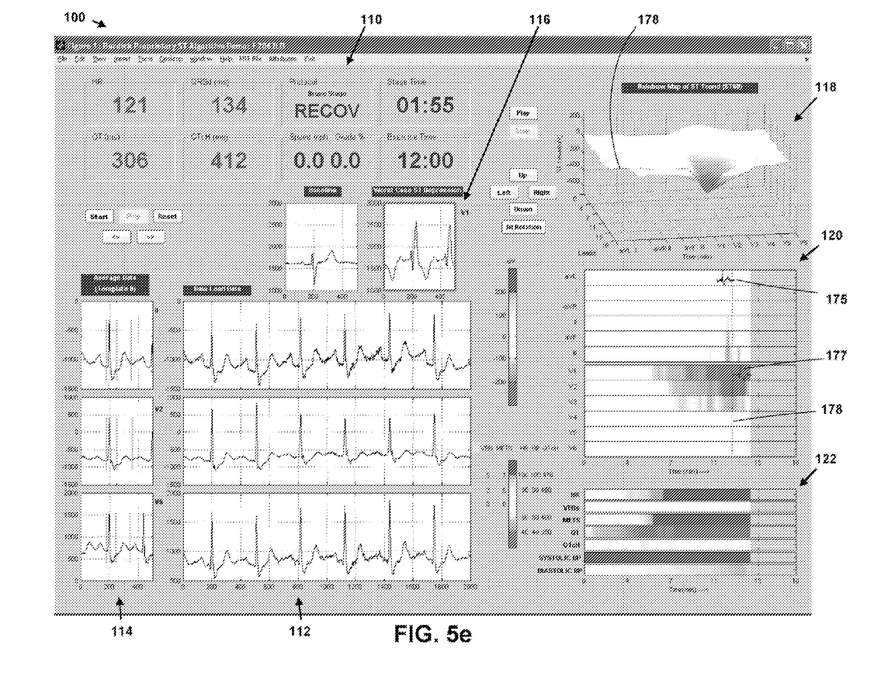

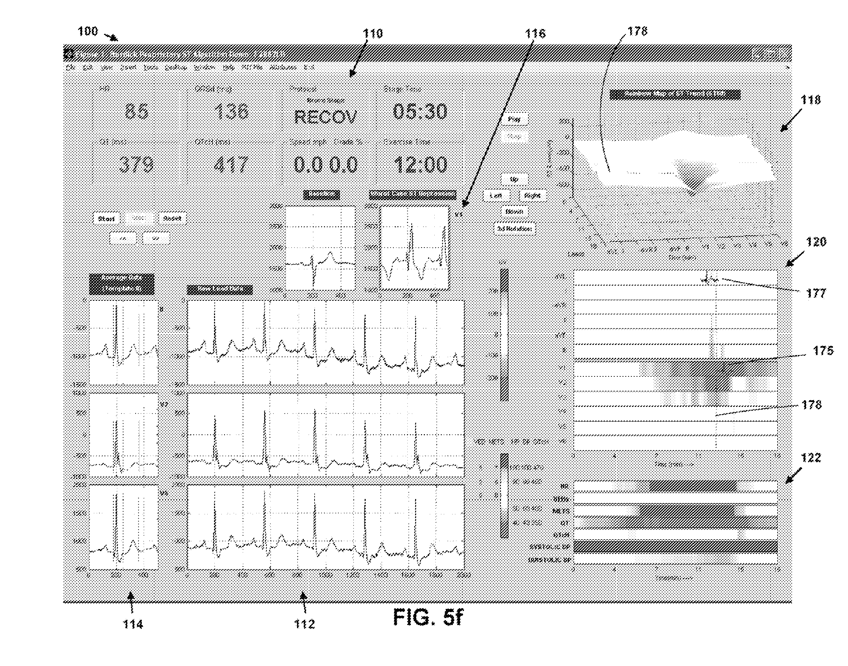

Figs. 5a-f show other examples of the color mapping interface display 100. The display contains a variety of components revealing data acquired during a patient stress test. The Figs. 5a-f are screen shots of the display taken at various times throughout the stress test and are set forth here in chronological order. The display is shown at intervals including: just after start in Fig. 5a; at just over 4 minutes into the test in Fig. 5b; about 8 minutes into the test at Fig. 5c; approximately 12 minutes into the test at Fig. 5d; approximately 14 minutes into the test at Fig. 5e; and at test completion at 18 minutes for Fig. 5f.

The basic components of the display are generally seen in each of the Figs. 5a-f. First, a measurement table 110 is found in the upper-left area of the graphic display window. The measurement table 110 is a measurement table which shows current heart rate 130, QT interval 132, averaged QRS duration 134, QTc value 136, stress protocol 138, speed and grade 140, stage time 142 and exercise time 144. The concise and prominent display of all of this information in an easy to locate area is helpful to medical personel seeking to monitor a patient with little more than a quick glance at times during the entire stress test protocol.

The display for the three channel real-time stress ECG traces 112 is located generally in the lower middle part of the window. The display shows three traces 112 of three user selected lead sets 146, 148, and 150 that may be chosen by the operator from the twelve measured data leads. Specifically, in the example of Figs. 5a-f, leads II, V2, and V5 are displayed, as can be recognized from the respective labels to the left of the traces 112. The display provides for a four second data display for each lead in the traces 112. When the "Start" button 151a or 151b is clicked on by the operator, the ECG signal continues to update and move from left to right as a real-time ECG monitoring display.

The three channel representative beat plots 114 are located in the lower-left of the window. The representative beat plots 114 have three user selected lead sets 152, 154, and 156 based on data selected from the twelve data leads having corresponding raw lead data shown in traces 112. These representative beat plots 114 provide for a one second data display for each lead. The data shows representative beats calculated from the past eight beats for each of the three leads. On each of the plots 152, 154, and 156 there are three vertical pink lines 169 on representative beats which represent, respectively, QRS onset 153, QRS offset 155, and T offset 157 points. The color of the frame surrounding each of the plots for each selected lead is changed corresponding to the 2-D ST color mapping at the specific time. This can be seen in Figs. 5c and 5d where the plot 154 for representative beat data of lead V2 is surrounded by a light blue border 158 or a dark blue border 159. These colors corresponding to the colors shown on the 2-D plot that time. It will help reviewers ensure the true-positive outcomes of the automatic detection algorithm.

The worst lead across twelve leads plot 116 is displayed near the middle of the window. In this plot, the representative beat of the worst lead across twelve leads with lowest ST depression is shown at Worst Case ST waveform 160 along with the lead name 162. The frame color of the worst lead is changed corresponding to the 2-D ST color mapping at the specific time. An example of this is seen in Figs. 5c and 5d where the plot 116 includes frames 165 and 167 bordered in different shades of blue for lead V1. Further, the baseline beat plot 164 is displayed to the left of the worst lead plot 116 generated from pretest stage 410 for comparison.

The 3-D color mapping plot 118 is displayed in the upper right hand corner of the display. The 3-D plot 118 shows ST level plotted over time for individualized leads including colorized scaling for a period beginning at pretest and lasting until the end of recovery, as determined by the stress test protocol. The X axis 166 represents leads, the Y axis 168 represents time, and the Z-axis 170 represents ST level. The 3-D plot is especially easy to read as its orientation can be manipulated by the user via controls at 172. The plot can be rotated around and looked at from different angles so as to give the user the desired perspective. Also, although the leads 166 are generally aligned in anatomic order, in some embodiments users may change this lead order to better suit his or her individual preferences. The 3-D plot is color coded and shares the same nonlinear color map meter 124 as the 2-D plot 120 found directly below it on the overall display. By viewing the 3-D plot 118, an operator can gauge the ST level change both in terms of 3-D topography as well as magnitude dictated by the color scheme scaling. The parameters used in the x and y axis can be switched or inverted by performing the appropriate rotation commands at the user's option.

Next, the 2-D color mapping plot 120 is located in the middle right of the window. It provides either a "bird's-eye" type view (from the top) or a "worm's eye" type view (from the bottom) of the 3-D plot 118. In Figs. 5a-f, a worm's eye view is shown. The 2-D plot 120 has color-coded ST level and user-selected lead order corresponding to 3-D plot 118. The X axis 174 represents time and the Y axis 176 represents leads. The color map meter 124 is on the left of the plot to define the scaling of various color values. By default, warm colors (i.e. red and yellow tones) are for ST elevation and cool colors (i.e. shades of blue tones) are for ST depression during a stress test. The program allows user changes so that the warm colors can be for ST derepression and cool colors for ST elevation. In addition to the color mapping, a small ST depression waveform 175 is found on the 2-D plot 120. The ST depression waveform 175 shows the worst case of ST depression during the stress test and shows when it occurred during the test. The small waveform 175 corresponds with the worst lead plot 116. Additionally, a small ST elevation waveform 177 is shown on the 2-D plot 120. This corresponds to the worst case ST elevation during a stress test. As can be observed in Figs. 5a-f, the location of these waveforms will likely continually change during the stress test. These two small waveforms superimposed on the time location of the worst cases colored areas help reviewers check true-positive outcomes from the automatic program. If a recovery start time is provided, a dotted line 178 is shown across twelve leads color mapping. For example, dotted line 178 can be seen in Figs. 5e and 5f. Both this 2-D plot 120 and the 3-D plot 118 can demonstrate exercise induced ischemia based on anatomic locations. The 3-D plot 118 is capable of showing how high the ST elevation rose or how low the ST depression fell across the particular anatomic positions and when ST changes were happening. Additionally, the 2-D plot 120 provides a clear and quick view of the ST changes. This 2-D plot 120 is especially useful when combined with the color map bars 122 based on other key parameters such as HR (heart rate) 180, number of VEBs (Ventricular Ectopic Beats) 182, METS (Metabloc Equivalent of Task) 184, QT interval 186, QTcH (Hodges corrected QT interval) 188, BP (systolic and diastolic blood pressures) 190, etc. These key parameters are presented in the lower right corner of the display for synthesis diagnosis and quick reference.

The key parameters color map bars 122 are located in the lower right of the window. Key parameters such as HR (heart rate) 180, number of VEBs (Ventricular Ectopic Beats) 182, METS (Metabolic Equivalent of Task) 184, QT interval 186, QTcH (Hodges corrected QT interval) 188, BP (systolic and diastolic blood pressures) 190, etc, are shown on color-coded bars. The key parameter color bars are all aligned by time to the 3-D and 2-D plots 118 and 120 to help with diagnosis. The 2-D plot 120 and color map bars 122 are in adjacent, close proximity and the time axis of these plots correspond horizontally with one another. This relationship allows a medical professional to quickly reference what is going on in terms of ST level in the 2-D plot 120 at any given time and then reference the data located vertically below this event to gain information regarding HR (heart rate) 180, number of VEBs 182, METS 184, QT interval 186, QTcH 188, and BP (systolic and diastolic blood pressures) 190 occurring at the same time of the event. Furthermore, a history of how these parameters changed throughout the stress test is quickly ascertainable. Note that the key parameters color map bars 122 have their own nonlinear color bar meter 126 to its left.

Color map meter 124 is used to gauge the ST change values. The instrument disclosed here uses colors to represent values where white means no ST changes, "warm" (yellow to red color) shows elevation, and "cold" (light to dark blue color) is used to represent depressions. The color map meter 124 of ST levels located on the middle right of the display represents the values within -200uV to 200uV (-2mm to 2mm). This range of ST level can be adapted as needed, however, this is the default arrangement as physicians do not usually attribute much added diagnostic difference for ST changes greater than 2mm. In some embodiments, a physician or user operator could adjust the range of the color meter as well, for individualized preference or convenience.

In the particular example shown in the 2-D plot 120 of Figs. 5a-f, regional clusters are shown where there was only a small amount of inferior ST depression (II , aVF, and III) twelve minutes into the test, Anteriorseptal ST depression (V1 to V3) starting around 7 minutes, and little or no Lateral ST elevation or depression (V5, V6, aVL, and I) throughout the entire test period shown. The recovery period is shown in Figures 5e and 5f at approximately 13 minutes by a dotted line 178 on both the 3-D plot 118 and the 2-D plot 120. As seen in Figure 5f, the waveform 175 representing the worst case ST depression occurred in lead V1 shortly after the recovery period began and the waveform 177 representing the worst case ST elevation occurred shortly before the recovery period in lead aVL. As seen here, the ST trend mapping with anatomic locations to position myocardial tissue ischemia/damage can be interpreted by medical personnel very quickly and easily.

In some embodiments, the user may be provided the option of changing lead configuration or sequence. For example, if a physician prefers a sequence as V6, V5, V4, I, II, III, aVF, aVL, aVR, V1, V2, and V3 rather than the standard sequence shown the order of the data plotted may be reordered. Furthermore, various embodiments allow 3-D mapping rotation such that a user can observe the best angle to view the changes versus time and anatomic locations.

During or after a stress test, medical professionals can quickly review the results. Just by locating the mouse arrow on an interesting point on the 2-D map 120, the corresponding raw channel data will be presented at 112 and the representative beats at 114.

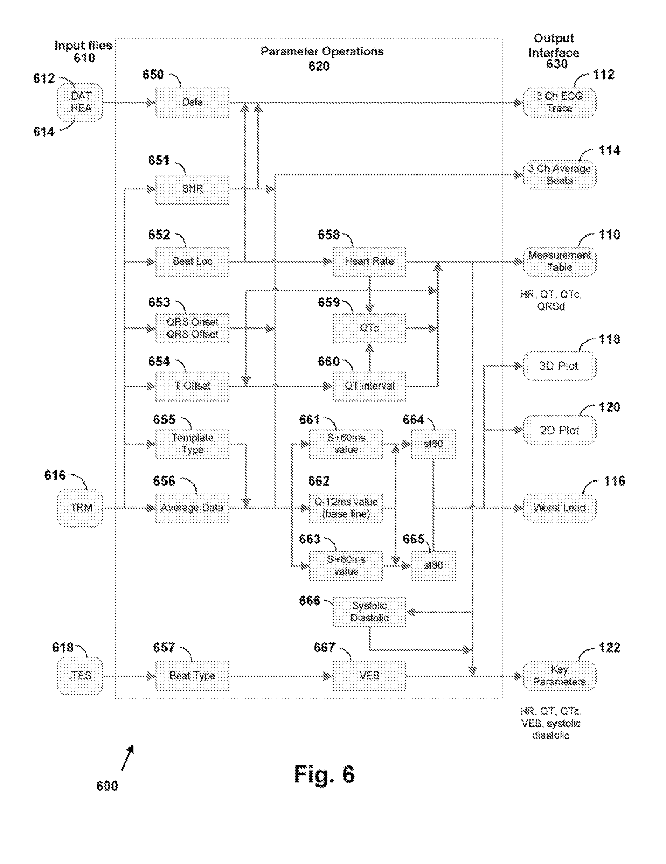

Fig. 6 is a block diagram setting forth an embodiment of the general data handling 600 which occurs based on the software used to generate the various outputs of the display 100. In general, input files 610 located on a data storage medium are acquired and input into the software for generating the display. In this embodiment, these input files 610 may include .DAT files 612, .HEA files 614, .TRM files 616, and .TES files 618. Parameter operations 620 are performed with the acquired input and data from these input files and undergo a variety of operations represented in Figure 6. Specifically, parameters and data values are manipulated to produce plots or maps and information for the output interface 630. The output display produced includes the three channel real-time stress ECG traces 112, the three channel average beat plots 114, the measurement table 110, the 3-D color mapping plot 118, the 2-D color mapping plot 120, the worst lead across twelve leads plot 116, and the key parameters color map bars 122.

As depicted in Fig. 6, input files 610 allow a number of first parameters to be read including data 650, SNR 651, beat loc 652, QRS onset/QRS offset 653, T offset 654, template type 655, average data 656 and beat type 657. Based upon these read parameters, further calculations of data can then be made using one or more of these values. Further, data calculations are represented in the data flow diagram of Fig. 6. Calculated values include Heart Rate 658, QTc 659, QT interval 660, S+60ms value 661, Q-12ms value (base line) 662, S+80 ms value 663, st60 664, st80 665, systolic and diastolic blood pressures 666, and VEB 667. Accordingly, the necessary data values are then made available for the various output interfaces 630 which display the various pieces of data in an intuitive manner as seen in Figures 5a-f.

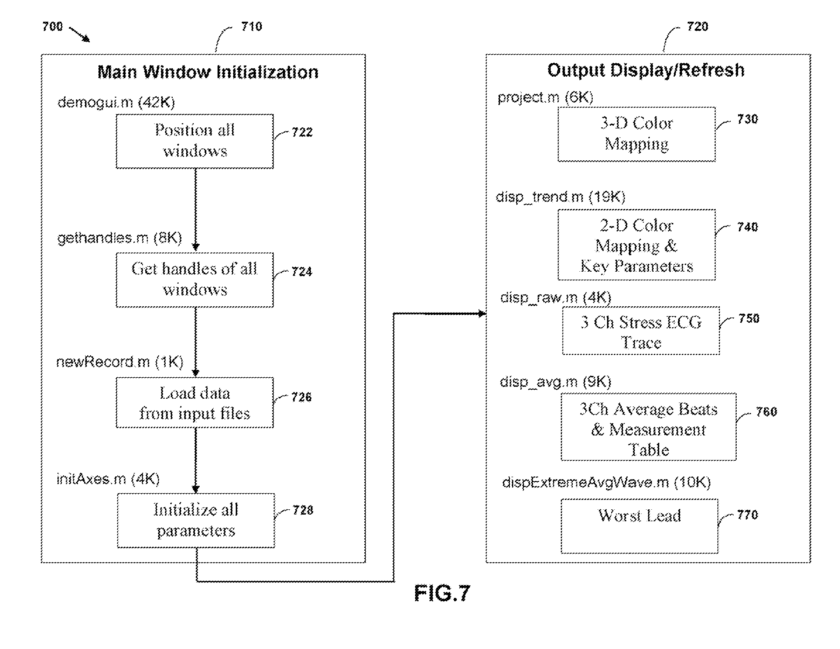

Fig. 7 is a flowchart that sets forth the manner in which the software sets the parameters and initializes data for display in the output window. This initialization and output process 700 generally involves a set of initialization operations 710 which are performed in sequence and a plurality of output files for various plots and information presented in the display 720. The first initialization operation file, ("demogui.m") involves positioning of the windows of the display at 722. Next, an operation file, ("gethandles.m") gets handles of all windows at 724. This is followed by loading data from the input files (from file "newRecord.m") via operation file at 726. This is followed by initialization of all parameters at 728 (using file initAxes.m). Output operations are next performed at 720. Output related to the 3-D plot 118 is generated at 730 (using file "project.m"). Output related to the 2-D color map plot 120 and Key Parameters color map bars 122 is generated at 740 (using file "disp_trend.m"). Output related to the three channel real-time stress ECG traces 112 is generated at 750 (using file "disp_raw.m"). Output related to the three channel average beat plots 114 and measurement table 110 is generated at 760 (using file "disp_avg.m"). Output related to the plot of the worst lead across twelve leads 116 is generated at 770 (using file "dispExtremeAvgWave.m").

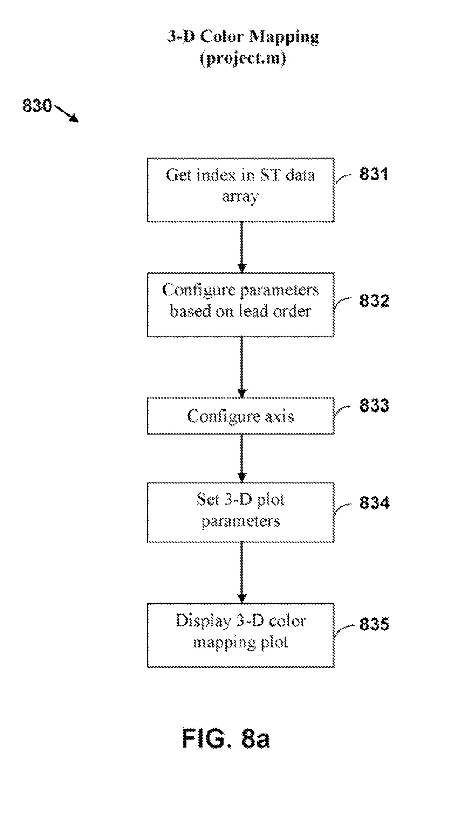

Figures 8a-e set forth the manner how each of the respective windows or displays containing plots, maps, charts or information set up parameters and is displayed. Figure 8a provides the steps 830 carried out in the code for the previously identified software file "project.m" for generating the 3-D plot 118 of the display. First at 831 the program gets the index in the ST data array. Next, the parameters are configured based upon lead order at 832. The axis is then configured at 833 followed by setting 3-D plot parameters at 834. Finally, the 3-D plot is displayed at 835.

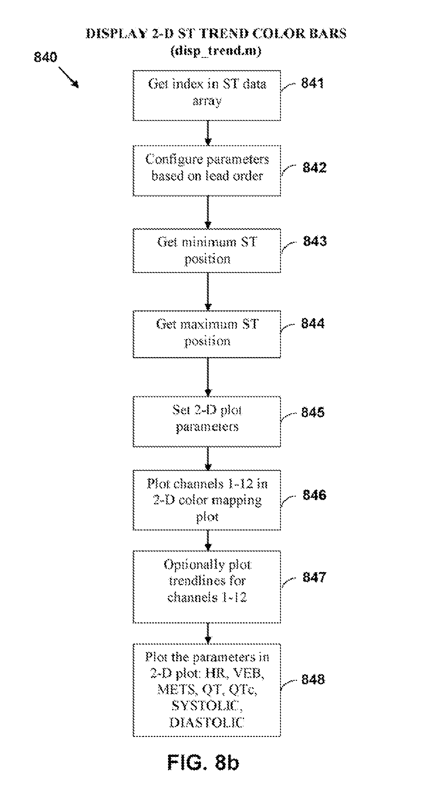

Figure 8b sets forth the steps 840 carried out by the software file "disp-trend.m" which is responsible for generating the 2-D plot 120 and key parameters color map bars 122. The steps include setting the index in the ST data array at 841, configuring parameters based upon the lead order at 842, getting minimum ST and maximum ST positions at 843 and 844, and setting the 2-D plot parameters at 845. Next, the twelve lead channels are plotted in a 2-D plot at 846 for the 2-D plot 120. Trendlines may be plotted for the twelve channels at 847 as well. Finally, parameters in the 2-D plot are plotted at 848, including HR, VEB, METs, QT, QTc, SYSTOLIC, and DIASTOLIC, which are used in the key parameters color map bars 122.

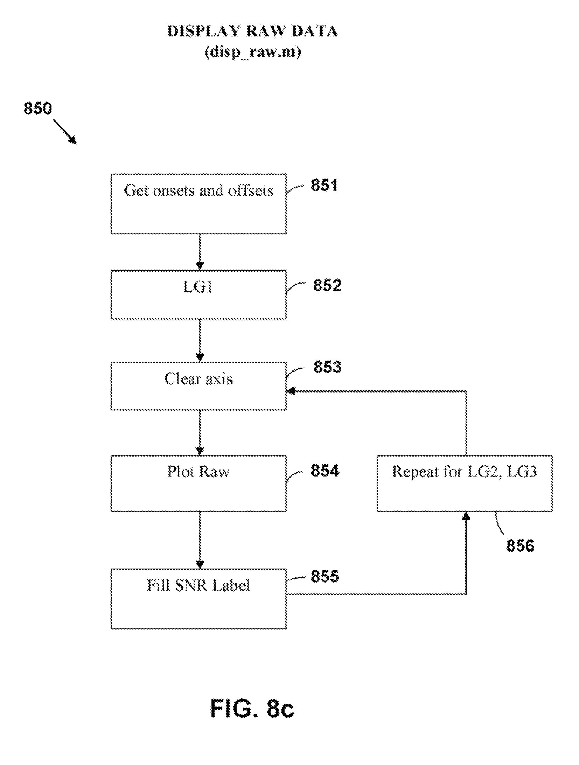

Figure 8c sets forth the steps 850 carried out by the software file "disp_raw.m" which is responsible for generating the three channel real-time stress ECG traces 112. First, the software gets the onsets and offsets at 851 for a first lead LG1 at 852. The axis are cleared at 853 followed by plotting raw lead data at 854 and filling the SNR label at 855. The steps of clearing the appropriate axis, plotting raw data and filling the SNR label may then be done for the data received for both leads LG2 and LG3 as well, as shown at 856.

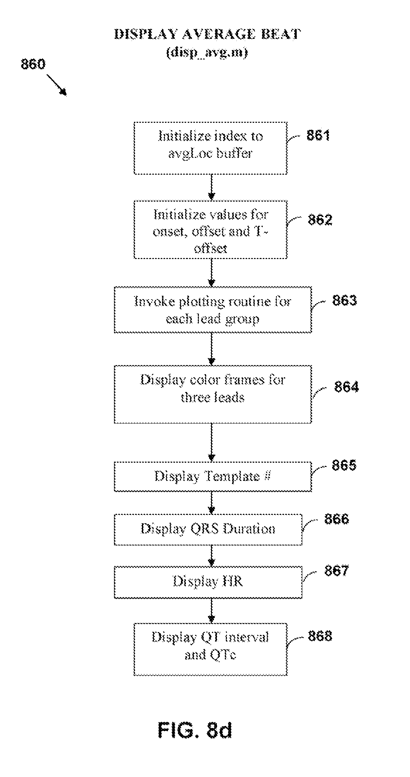

Figure 8d sets forth steps 860 for the software file "disp_avg.m" which generates the three channel average beat plots 114 and measurement table 110. First, at 861 the index is initialized to the avgLoc buffer. Next, the values for onset, offset and T-offset are initialized at 862 and a plotting routine is invoked for each lead group at 863. Color frames are then displayed for three leads at 864 at respective plots 152, 154, and 156. Additionally the template number 865, QRS duration 866, HR 867, and QT interval and QTc at 868 are all displayed. These values can thereafter be found in respective locations throughout the measurement table 110.

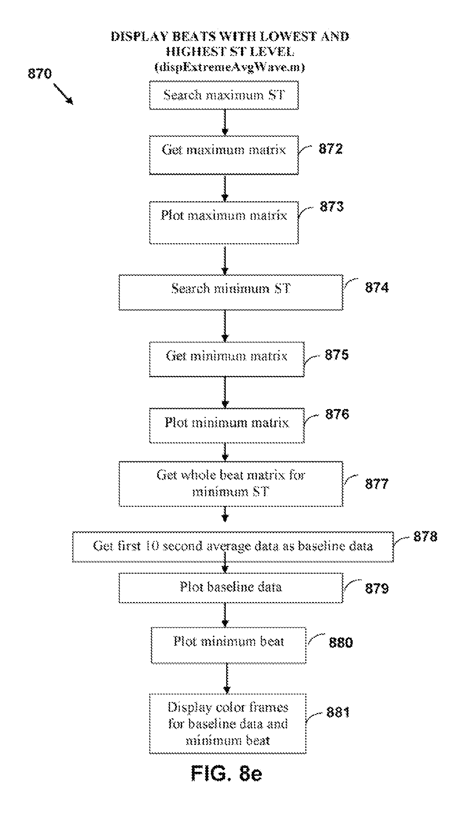

Figure 8e sets forth steps 870 for the software file "dispExtremeAvgWave.m". This file is responsible for generating the worst lead across the twelve leads for display plot 116. First, a search is done to obtain the maximum ST at 871. Next, a maximum matrix is obtained at 872 and is plotted at 873. A search for a minimum ST is done to obtain the minimum ST at 874. Next, a minimum matrix is obtained at 875 and is plotted at 876. The whole beat matrix for minimum ST is obtained at 877. Next, the first ten second average data as baseline data is obtained at 878. Baseline data is plotted at 879, minimum beat is plotted at 880 and the color frames for baseline data and minimum beat are displayed at 881.

An example of the MATLAB source code used to generate the display is included on the compact disc included with this application. The file names and routines referenced in the application specification and figures correspond to those of the included MATLAB source code. The description of the MATLAB code used to construct the display is not intended to be limiting of the manner of generating or carrying out the described display. Similar displays may be produced using various other programming languages or tools.

Applications to this product extend beyond typical stress testing products, chemical stress or electrical stress testing products, or management system products. The display may have applications in holter, monitoring, and ECG. Additionally, echo cardiology applications are possible as well.

Applications in stress tests and other types of tests may make use of less than the standard twelve lead ECG configuration. As long as it is known what the leads are and that there is more than just a single lead, the display can be readily modified to display fewer leads and to appropriately build a 2-D/3-D plot. This display can be applied to twelve lead, or even two-/three- lead Holter products, cardiac patent monitors, and telemetry even though the leads are often placed in arbitrary locations.

The embodiments above are intended to be illustrative and not limiting. Additional embodiments are within the claims. Although the present invention has been described with reference to particular embodiments, workers skilled in the art will recognize that changes may be made in form and detail without departing from the spirit and scope of the invention.

Various modifications to the invention may be apparent to one of skill in the art upon reading this disclosure. For example, persons of ordinary skill in the relevant art will recognize that the various features described for the different embodiments of the invention can be suitably combined, un-combined, and re-combined with other features, alone, or in different combinations, within the spirit of the invention. Likewise, the various features described above should all be regarded as example embodiments, rather than limitations to the scope or spirit of the invention. Therefore, the above is not contemplated to limit the scope of the present invention.

For purposes of interpreting the claims for the present invention, it is expressly intended that the provisions of Section 112, sixth paragraph of 35 U.S.C. are not to be invoked unless the specific terms "means for" or "step for" are recited in a claim.

## Claims

1. A computer-implemented method for displaying data of a human heart for analysis, comprising the steps of:
providing a display for presenting information obtained during a cardiac stress
test;
receiving data, wherein the data is cardiac data produced during the cardiac stress
test by a plurality of electrode leads adapted for placement on a patient;
calculating values for use in plots based on said data;
displaying a three dimensional color plot in a first portion of the display based on
the data and the calculated values;
displaying a two dimensional color plot in a second portion of the display based
on the data and the calculated values, said two dimensional color plot
corresponding to the three dimensional color plot; and
displaying a plot of raw lead data from at least one lead in a third portion of the
display.

2. The computer-implemented method of claim 1, wherein the cardiac data is cardiac electrical impulse data and the calculated values are electrocardiography values.

3. The computer-implemented method of claim 1 or 2, wherein the three dimensional color plot may be rotated on the display such that it may be observed from a desired viewpoint and/or wherein the lead data is shown on the two dimensional color plot and three dimensional color plot in anatomic lead order.

4. The computer-implemented method of one of the above claims, wherein the lead data is shown on the two dimensional color plot and the three dimensional color plot in an order which is user selectable and/or wherein the three dimensional color plot is located directly above the two dimensional color plot.

5. The computer-implemented method of one of the above claims, wherein said two dimensional color plot corresponding to the three dimensional color plot in colors used to depict ST level; and wherein in particular
the plot of raw lead data from at least one lead located is displayed near the center of the display.

6. The computer-implemented method of one of the above claims, wherein the cardiac data is acquired by measuring the electric potential between various points of the body using a data acquisition module and a plurality of electrode leads adapted for patient attachment;
generating electrocardiogram information for each of the plurality of electrode leads based upon said data;
said calculated values are ST level data for each of the plurality of electrode leads throughout the stress test;
displaying the three dimensional color plot using a first color scale where the ST level data are represented by color and amplitude for the plurality of electrode leads;
displaying the two dimensional color plot using the first color scale where ST level data is represented by color for the plurality of electrode leads;
displaying a two dimensional color map of various electrocardiogram parameters based upon a second color scale, the two dimensional color map located adjacent to the two dimensional color plot such that the two dimensional color plot and the two dimensional color map are positioned to correspond horizontally by time.

7. The computer-implemented method of one of the above claims, wherein
said received data is received from one or more input files,
providing said display in such a way that a plurality of display windows are positioned within said display;
displaying said three dimensional color plot in one of the plurality of display windows;
displaying said two dimensional color plot in one of the plurality of display windows, the two dimensional color plot and the three dimensional color plot correspond with one another and use color to represent ST level.

8. The computer-implemented method of claim 7, wherein the color shown in the two dimensional color plot and the three dimensional color plot represents ST level and/or further including a two dimensional color map located adj acent to the two dimensional color plot on the display, the two dimensional color map having an independent color scale.

9. The computer-implemented method of one of the above claims, further including generating a printable one page stress test report containing the information contained on the display and/or further including displaying a plot of raw channel data and a representative beat plot in the display corresponding to a user selected location on the two dimensional plot.

10. A medical apparatus for generating a stress test display, comprising
a data acquisition module adapted for connection to a patient undergoing a cardiac stress test;
a monitor capable of presenting a real time display of color mapped plots;
a computer, comprising at least a processor and a memory, coupled to the data acquisition module and the monitor, said computer containing exercise stress test software, operation of the software including:
means for providing a display for reviewing information obtained during a cardiac
stress test;
means for receiving data, wherein the data is cardiac data produced during the
cardiac stress test by a plurality of electrode leads adapted for placement
on a patient;
means for calculating values for generating plots based on said data;
means for displaying a three dimensional color plot in a first portion of the display
based on the data and the values;
means for displaying a two dimensional color plot in a second portion of the
display based on the data and the values, said two dimensional color plot
corresponding to the three dimensional color plot; and
means for displaying a plot of raw lead data from at least one lead in a third
portion of the display.

11. The medical apparatus of claim 10, wherein the three dimensional color plot may be rotated on the display such that it may be observed from a desired viewpoint and/or wherein the lead data is shown on the two dimensional color plot and three dimensional color plot in anatomic lead order.

12. The medical apparatus of claim 10 or 11, wherein the lead data is shown on the two dimensional color plot and three dimensional color plot in an order which is user selectable and/or wherein the first portion is located vertically adjacent to the second portion of the display.

13. The medical apparatus of one of claims 10 to 12, comprising
a treadmill for patient use during the course of a cardiac stress test; and
a plurality of electrode leads adapted for patient attachment during the cardiac stress test;
wherein said data acquisition module is adapted for connection to the electrode leads for measuring electrical potential between various body locations on said patient.

14. The medical apparatus of one of claims 10 to 13, wherein said received data is cardiac electrical impulse data.

15. A computer program product, which comprises software code in order to carry out the following steps, when the product is executed,
providing a display of information obtained during the course of a cardiac stress test;
receiving cardiac electrical impulse data for the cardiac stress test, wherein the data is obtained from a data acquisition module connected to electrode leads;
calculating values for generating plots based on said cardiac electrical impulse data;
displaying a three dimensional color plot in a first portion of the display based on the cardiac electrical impulse data and the values;
displaying a two dimensional color plot in a second portion of the display based on the cardiac electrical impulse data and the values, said two dimensional color plot corresponding to the three dimensional color plot.

16. The computer program product of claim 15, which comprises software code in order to carry out the following steps, when the product is executed, displaying a two dimensional color map of various electrocardiogram parameters, the two dimensional color map located adjacent to the two dimensional color plot such that the two dimensional color plot and the two dimensional color map are positioned to correspond horizontally by time.

17. A computer readable medium comprising the software code of claim 15 or 16.
